# EUROPÄISCHE PATENTANMELDUNG

(11) **EP 4 748 405 A2**
(43) Veröffentlichungstag der Anmeldung: **27.05.2026**
(21) Anmeldenummer: 25216501.4
(22) Anmeldetag: 18.11.2025
(51) Int. Cl.: A61L 9/12

(54) **VORRICHTUNG ZUR ABGABE EINES WIRKSTOFFES**

(30) Priorität: 21.11.2024 AT 509242024
(71) Anmelder: Lechner, Martin, 6114 Kolsass (AT)
(72) Erfinder: Lechner, Martin, 6114 Kolsass (AT)
(74) Vertreter: Torggler & Hofmann Patentanwälte - Innsbruck

(57) **Zusammenfassung**

Vorrichtung zur Abgabe eines Wirkstoffes, insbesondere eines Duftstoffes, umfassend
- eine Aufnahmevorrichtung (2) zur Befestigung der Vorrichtung (1) an einer Öffnung (5) eines Behältnisses (3), vorzugsweise einer Flasche, zur Aufnahme des Wirkstoffes,
- eine Zuführvorrichtung (4), welche Zuführvorrichtung (4) dazu ausgebildet ist, einen Trägergasstrom (30), vorzugsweise einen Luftstrom, über die Öffnung (5) des Behältnisses (3) in das Behältnis (3) zu leiten, und
- eine Abführvorrichtung (6), welche Abführvorrichtung (6) dazu ausgebildet ist, ein mit Wirkstoff beladenen Trägergasstrom (30) über die Öffnung (5) des Behältnisses (3) aus dem Behältnis (3) abzuführen,
wobei wenigstens eine mit einem Aktuator (7) verbundene Blende (8) vorgesehen ist, wobei durch den Aktuator (7) und die damit verbundene wenigstens eine Blende (8) ein Volumenstrom des Trägergasstroms (30) durch die Zuführvorrichtung (4) und/oder die Abführvorrichtung (6) veränderbar ist.

## Beschreibung

Die vorliegende Erfindung betrifft eine Vorrichtung zur Abgabe eines Wirkstoffes, insbesondere eines Duftstoffes, mit dem Merkmal des Oberbegriffs des Anspruches 1.

Gattungsgemäße Vorrichtungen zur Abgabe von Wirkstoffen umfassen
- eine Aufnahmevorrichtung zur Befestigung der Vorrichtung an einer Öffnung eines Behältnisses, vorzugsweise einer Flasche, zur Aufnahme des Wirkstoffes,
- eine Zuführvorrichtung, welche Zuführvorrichtung dazu ausgebildet ist, einen Trägergasstrom, vorzugsweise einen Luftstrom, über die Öffnung des Behältnisses in das Behältnis zu leiten, und
- eine Abführvorrichtung, welche Abführvorrichtung dazu ausgebildet ist, einen mit Wirkstoff beladenen Trägergasstrom über die Öffnung des Behältnisses aus dem Behältnis abzuführen.

Aus dem Stand der Technik bekannte Vorrichtungen zur Abgabe eines Wirkstoffes sind zumeist passiv ausgebildet, wobei durch die Vorrichtung ein Wirkstoff, beispielsweise ein Duftstoff, aus einem Behältnis gegenüber der Umgebung freigegeben wird, wodurch der Wirkstoff verdunstet oder verdampft und somit in der Umgebung verteilt wird.

Hierfür wird ein Luftaustausch oder eine Strömung der Umgebung genutzt, welche zur Verbreitung des Wirkstoffes genutzt wird.

Entsprechende Vorrichtungen des Stands der Technik sind beispielsweise durch die CN 210785581 U, die WO 2022/261884 A1, die CN 215134046 U oder die CN 216755071 U gezeigt.

Diese Vorrichtungen umfassen einen Basisbehälter, in welchem der Wirkstoff, zumeist ein Duftstoff, angeordnet werden kann. Der Basisbehälter umfasst entlang seines Umfanges Lüftungsschlitze, welche es der Umgebungsluft erlauben, in den Behälter ein- und auszutreten. Beim Durchtreten der Vorrichtung durch die Umgebungsluft wird durch Verdampfung oder Verdunstung der Luft Wirkstoff beigemengt. Um die Intensität der Freisetzung des Wirkstoffes regulieren zu können, können die Belüftungsschlitze zumindest teilweise abgedeckt werden.

Nachteilig an den bekannten Vorrichtungen zur Abgabe eines Wirkstoffes ist jedoch, dass sich keine zielgerichtete, genaue Abgabe des Wirkstoffes, insbesondere des Duftstoffes, einstellen lässt.

So ist die Intensität der Abgabe des Wirkstoffes stark abhängig von Umgebungseinflüssen und der Luftbewegung der Umgebung.

Des Weiteren lässt sich kaum zielgerichtet auf den Wirkstoff eine Einstellung vornehmen. Was jedoch wünschenswert wäre, da je nach Wirkstoff oder Duftstoff und dessen Intensität mehr oder weniger Wirkstoff an die Umgebung abgegeben werden möchte, sodass nicht ein unangenehmes Gefühl für Zielpersonen in der Umgebung ergibt, da zu viel Wirkstoff in der Umgebungsluft getragen wird.

Ein weiterer Nachteil besteht darin, dass sich die Vorrichtungen nicht auf die verändernde Menge an Wirkstoff in der Vorrichtung einstellen lassen. So sinkt die Intensität des abgegebenen Wirkstoffes mit sinkenden Wirkstoffgehalt in der Vorrichtung, womit eine kontinuierliche Betreuung der Vorrichtung notwendig ist, um eine kontinuierliche Abgabe an Wirkstoff umsetzen zu können.

Jedoch ist gerade bei Anwendungen im öffentlichen Raum, Restaurants, Praxen oder Hotels, nur ein geringer Wartungsaufwand der Vorrichtung möglich, womit der Wunsch hinsichtlich einer Vorrichtung zur Abgabe eines Wirkstoffes besteht, welche eine kontinuierliche Menge an Wirkstoff über einen möglichst langen Zeitraum (Monate oder sogar Jahre) abgeben kann, ohne dass ein Bedienpersonal nachjustieren oder neu befüllen muss.

Aufgabe der vorliegenden Erfindung ist es, eine Vorrichtung zur Abgabe eines Wirkstoffes, insbesondere eines Duftstoffes, bereitzustellen, mit welcher die zuvor beschriebenen Nachteile des Standes der Technik zumindest teilweise verbessert werden können und/oder die Abgabe eines Wirkstoffes verbessert werden kann und/oder eine effizientere Nutzung des Wirkstoffes umgesetzt werden kann und/oder eine genauere Dosierung des Wirkstoffes bewerkstelligt werden kann und/oder welche Vorrichtung einen geringeren Wartungsaufwand erfordert.

Diese Aufgabe wird durch eine Vorrichtung zur Abgabe eines Wirkstoffes, insbesondere eines Duftstoffes, mit dem Merkmal des Anspruches 1, mit einer Anordnung einer solchen Vorrichtung und einem Behältnis zur Aufnahme des Wirkstoffes, sowie einer Gebäudebelüftungsvorrichtung mit einer entsprechenden Vorrichtung gelöst.

Eine Vorrichtung zur Abgabe eines Wirkstoffes, insbesondere eines Duftstoffes, gemäß der vorliegenden Offenbarung, umfasst
- eine Aufnahmevorrichtung zur Befestigung der Vorrichtung an einer Öffnung eines Behältnisses, vorzugsweise einer Flasche, zur Aufnahme des Wirkstoffes,
- eine Zuführvorrichtung, welche Zuführvorrichtung dazu ausgebildet ist, einen Trägergasstrom, vorzugsweise einen Luftstrom, über die Öffnung des Behältnisses in das Behältnis zu leiten, und
- eine Abführvorrichtung, welche Abführvorrichtung dazu ausgebildet ist, einen mit Wirkstoff beladenen Trägergasstrom über die Öffnung des Behältnisses aus dem Behältnis abzuführen,
wobei wenigstens eine mit einem Aktuator verbundene Blende vorgesehen ist, wobei durch den Aktuator und die damit verbundene wenigstens eine Blende ein Volumenstrom des Trägergasstroms durch die Zuführvorrichtung und/oder die Abführvorrichtung veränderbar ist.

Durch das separierte Vorsehen einer Zuführvorrichtung, sowie einer Abführvorrichtung, kann zielgerichtet ein Trägergasstrom in das Behältnis, bzw. auf eine Oberfläche des Wirkstoffes im Behältnis, gerichtet und aus dem Behältnis wieder abgeführt werden, wodurch sich eine Zirkulation des Trägergasstromes im Behältnis steuern lässt und die Beladung des Trägergasstromes mit Wirkstoff reproduzierbar steuern lässt.

Die Abgabe des Wirkstoffes an den Trägergasstrom kann durch Verdampfung und/oder Verdunstung erfolgen, wobei der Wirkstoff im Trägergasstrom aufgenommen wird. Grundsätzlich wäre es auch denkbar, dass der Wirkstoff in Form eines Aerosols im Trägergasstrom vorliegt.

Durch das Vorsehen der Blende, welche mit einem Aktuator verbunden ist, kann der Volumenstrom des Trägergasstromes eingestellt, gesteuert oder geregelt werden, wodurch Einfluss auf den Austrag des Wirkstoffes genommen werden kann.

So kann beispielsweise über den Aktuator und die Blende ein Volumenstrom durch das Behältnis verringert werden, wenn es sich um einen Wirkstoff handelt, welcher nur in einem geringen Maß an den Trägergasstrom abgegeben werden soll.

Weiters ist die Möglichkeit geschaffen, dass über den Aktuator automatisiert, die Blende im Laufe der Zeit den Volumenstrom des Trägergasstromes erhöht, sodass mit sinkendem Wirkstoff im Behältnis, der Trägergasstrom erhöht wird, um eine trotz sinkenden Wirkstoffvorrats kontinuierlicher Austrag des Wirkstoffes sicher zu stellen.

Weiters wird die Möglichkeit geschaffen, durch automatisierte Steuer- oder Regelung des Aktuators eine Vorrichtung mit verringerter Wartungsintensität bereit zu stellen, welche keine kontinuierliche Anpassung, Wartung, und/oder Befüllung der Vorrichtung erfordert.

Folglich kann mit einer Vorrichtung gemäß der vorliegenden Offenbarung eine im Wesentlichen kontinuierliche Abgabe eines Wirkstoffes erreicht werden und/oder - vorzugsweise jederzeit - eine genauere Dosierung des Wirkstoffes bewerkstelligt werden.

Als Wirkstoff können beispielsweise Dufte, Parfüms, Medikamente, Inhalator, geruchsneutralisierende Stoffe oder der ähnlichen ihre Anwendung finden.

Die Vorrichtung kann auch in bereits bekannten Ausführungsvarianten des Standes der Technik, wie beispielsweise in der Beschreibungseinleitung beschrieben, ihren Einsatz finden und nachträglich installiert werden.

Die Abgabe kann eine Verdunstung- und/oder Verdampfung des Wirkstoffes einschließen, wobei der verdunstete und/oder verdampfte Wirkstoff in den Trägergasstrom abgegeben und von diesem aufgenommen oder getragen wird. Analoges gilt für die erwähnte Option mit einem Aerosol.

Das Behältnis ist zur Aufnahme des Wirkstoffs geeignet und/oder ausgebildet.

Vorteilhafte Ausführungsformen der Erfindung sind anhand der abhängigen Ansprüche definiert.

Es kann vorgesehen sein, dass wenigsten eine Fördervorrichtung zum Erzeugen des Trägergasstroms über die Zuführvorrichtung in das Behältnis vorgesehen ist, vorzugsweise wobei die wenigstens eine Fördervorrichtung zumindest einen Ventilator umfasst, besonders bevorzugt welche in Strömungsrichtung stromaufwärts der Zuführvorrichtung angeordnet ist.

Die Abgabe des Wirkstoffes kann durch die Fördervorrichtung und die dadurch hervorgerufene Trägergaszirkulation im Behältnis beeinflusst werden, wodurch eine kontinuierliche Ausbringung des Wirkstoffes hervorgerufen werden kann, unabhängig von einem Speicherzustand des Behältnisses und/oder Strömungsbedingungen der Umgebung.

Die Fördervorrichtung kann dazu ausgebildet sein, eine Bewegung, Strömung und/oder Zirkulation des Trägergases hervorzurufen und/oder zu fördern.

Vorzugsweise kann vorgesehen sein, dass die Zuführvorrichtung einen Anströmbereich aufweist, welcher - vorzugsweise trichterförmig ausgebildete - Anströmbereich dazu ausgebildet ist, den Trägergasstrom zu erfassen und fokussiert als Strahl über die Öffnung des Behältnisses in das Behältnis einzuleiten.

Es kann vorgesehen sein, dass die Zuführvorrichtung zumindest eine Leiteinrichtung aufweist, welche zumindest eine Leiteinrichtung dazu ausgebildet ist, den in den Behälter eingeleiteten Trägergasstrom auf einen Wirkstoffpegel im Behältnis zu leiten.

Vorzugsweise kann vorgesehen sein, dass die zumindest eine Leitvorrichtung ein Leitelement, vorzugsweise ein Leitblech, aufweist, welches Leitelement an der Aufnahmevorrichtung hervorsteht, sodass das Leitelement in einem Befestigungszustand der Vorrichtung an dem den Wirkstoff tragenden Behältnis über die Öffnung des Behältnisses in das Innere des Behältnisses ragt.

Es kann vorgesehen sein, dass die wenigstens eine Blende als Lochscheibe ausgebildet ist, welche Lochscheibe durch eine oder eine Vielzahl an Öffnungen - vorzugsweise Bohrungen mit unterschiedlichen Durchmessern - dazu ausgebildet ist, einen Strömungsquerschnitt eines den Trägergasstrom leitenden Strömungskanals der Zuführvorrichtung und/oder der Abführvorrichtung, je nach Relativstellung der Lochscheibe zum Strömungskanal, zu variieren.

Vorzugsweise kann vorgesehen sein, dass die Lochscheibe drehbar um eine Rotationsachse gelagert ist und der, vorzugsweise als rotatorischer Motor, besonders bevorzugt rotatorischer Elektromotor, ausgebildete, Aktuator dazu ausgebildet ist, die Lochscheibe rotatorisch um die Rotationsachse anzutreiben.

Es kann vorgesehen sein, dass die Vorrichtung einen, vorzugsweise eine Mittelachse aufweisenden zylinderförmigen, Basiskörper aufweist, welcher Basiskörper:
- einen ersten Strömungskanal der Zuführvorrichtung aufweist, vorzugsweise welcher erste Strömungskanal der Zuführvorrichtung den Basiskörper parallel zur Mittelachse durchdringt, und/oder
- einen zweiten Strömungskanal der Abführvorrichtung aufweist, vorzugweise welcher zweite Strömungskanal der Abführvorrichtung eine Öffnung an einer Stirnseite des Basiskörpers mit einer Öffnung an einer Zylinderfläche des Basiskörpers (verbindet, und/oder
- einstückig mit der Aufnahmevorrichtung zur Befestigung der Vorrichtung an einer Öffnung eines Behältnisses, vorzugsweise an einer Stirnseite des Basiskörpers, ausgebildet ist, und/oder
- dazu ausgebildet ist, die wenigstens eine Blende aufzunehmen, und vorzugsweise die wenigstens eine Blende rotatorisch zu lagern, und/oder
- dazu ausgebildet ist, den Aktuator aufzunehmen und/oder zu lagern, und/oder
wenigstens eine Befestigungsvorrichtung zur Befestigung der Vorrichtung an einem externen Objekt aufweist.

Es kann vorgesehen sein, dass eine mit dem Aktuator signalleitend verbundene Steuer- oder Regelvorrichtung vorgesehen ist, welche dazu ausgebildet ist, durch Steuern oder Regeln des Aktuators den Volumenstrom des Trägergasstroms durch die Zuführvorrichtung und/oder die Abführvorrichtung zu variieren.

Es kann vorgesehen sein, dass die Steuer- oder Regelvorrichtung mit wenigstens einem Sensor signalleitend verbunden ist, wobei der wenigstens eine Sensor dazu ausgebildet ist, ein für eine Beladung des Trägergasstroms mit Wirkstoff charakteristisches Signal auszugeben, wobei die Steuer- oder Regelvorrichtung dazu ausgebildet ist, dieses charakteristische Signal mit einem hinterlegten oder hinterlegbaren Grenzwert zu vergleichen und bei Feststellen einer Abweichung eine entsprechende Anpassung des Volumenstroms vorzunehmen.

In anderen Worten ausgedrückt, kann es vorgesehen sein, dass in der Steuer- oder Regelvorrichtung ein gewisser Grenzwert für die Beladung des Trägergases mit einem Wirkstoff hinterlegt ist, wobei über den wenigstens einen Sensor und dessen charakteristisches Signal der aktuell vorliegende Beladungszustand des Trägergases überwacht werden kann und je nach Abweichung die Steuer- oder Regelvorrichtung, den Aktuator dermaßen ansteuern oder regeln kann, um eine vorliegende Abweichung der Beladung des Trägergases mit Wirkstoff an den hinterlegten Grenzwert anzunähern.

Es kann vorgesehen sein, dass eine tolerierte Abweichung vom Grenzwert vorgesehen ist, innerhalb welcher die tatsächliche Beladung des Trägergasstromes von einem Grenzwert abweichen kann, ohne dass eine Anpassung vorgenommen wird.

Es kann vorgesehen sein, dass die Steuer- oder Regelvorrichtung dazu ausgebildet ist, nach einer vorgebbaren Zeit oder einem Zeitintervall den Volumenstrom des Trägergases über die Blende oder den Aktuator anzupassen. Somit kann vorgesehen sein, dass die Steuer- oder Regelvorrichtung automatisch in Zeitintervallen den Volumenstrom des Trägergases durch die Vorrichtung anpasst, um auf einen sinkenden Pegel des Wirkstoffes im Behältnis zu reagieren.

Vorzugsweise kann vorgesehen sein, dass die Steuer- oder Regelvorrichtung eine Eingabeschnittstelle aufweist oder mit einer solchen signalleitend verbunden ist, wobei die Steuer- oder Regelvorrichtung dazu ausgebildet ist, eine Anpassung des Volumenstroms unter Berücksichtigung eines über die Eingabeschnittstelle bereitgestellten Signals vorzunehmen, welches Signal mittels der Eingabeschnittstelle über eine Datenübertragungsverbindung entgegengenommen werden kann.

Die Datenübertragungsverbindung kann bevorzugt als Datenfernübertragungsverbindung ausgeführt sein. Die Datenfernübertragungsverbindung kann mittels einer LAN (Local Area Network), WLAN (Wireless Local Area Network), WAN (Wide Area Network, Bluetooth-Verbindung und/oder verschiedener (Internet-) Protokolle realisiert sein.

So könnte es beispielsweise vorgesehen sein, dass der Steuer- oder Redevorrichtung ein Signal über eine App, vorzugsweise eine Handy-App oder Smartphone-APP, an der Eingabeschnittstelle bereitstellbar ist, sodass über die App Anpassungen hinsichtlich des Volumenstroms über die Steuer- oder Regelvorrichtung und den Aktuator vorgenommen werden können.

Es kann vorgesehen sein, dass die Vorrichtung ein Sicherungsventil umfasst, welches Sicherungsventil dazu ausgebildet ist ein Inneres des Behältnisses oder der Vorrichtung zur Belüftung mit der Umgebung zu verbinden. Wenn beispielsweise Wirkstoffe Anwendung finden, welche Brennbare Eigenschaften aufweisen, könnte es bei einer bestimmten Menge an Sauerstoff und Wirkstoff im Behältnis zu einer explosiven Atmosphäre im Behältnis führen, wobei über ein Sicherheitsventil zusätzlich Umgebungsluft zur Belüftung des Behältnisses zugeführt werden kann, um eine entsprechende explosiven Atmosphäre zu verhindern.

Weiters wird Schutz begehrt für eine Anordnung einer entsprechenden Vorrichtung und einem Behältnis zur Aufnahme des Wirkstoffes, wobei die Vorrichtung über die Aufnahmevorrichtung an einer Öffnung des Behältnisses befestigt ist.

Ebenfalls wird Schutz begehrt, für eine Gebäudebelüftungsvorrichtung, umfassend eine entsprechende Vorrichtung gemäß einem Ausführungsbeispiel der vorliegenden Offenbarungen.

Es kann vorgesehen sein, dass eine Vorrichtung gemäß der vorliegenden Offenbarung in eine Gebäudebelüftungsvorrichtung eingesetzt ist, wobei ein bereits durch Gebäudebelüftungsvorrichtung erzeugter Luftstrom als Trägergasstrom genutzt wird und von der Zuführvorrichtung der Vorrichtung zur Abgabe eines Wirkstoffes in die Vorrichtung eingeleitet wird.

Weitere Vorteile und Einzelheiten ergeben sich aus den Figuren, sowie der dazugehörigen Figurenbeschreibung. Dabei zeigt:
- Fig. 1: ein erstes Ausführungsbeispiel einer Vorrichtung zur Abgabe eines Wirkstoffes,
- Fig. 2: einen Querschnitt durch das Ausführungsbeispiel der Fig. 1,
- Fig. 3: eine Explosionsdarstellung des Ausführungsbeispiels der Fig. 1,
- Fig. 4: die Explosionsdarstellung der Fig. 3 mit aufgeklappten Einzelteilen,
- Fig. 5: eine Detailansicht der Blende aus den vorhergehenden Figuren,
- Fig. 6+7: eine perspektivische Detailansicht des Basiskörpers aus den vorhergehenden Figuren,
- Fig. 8: ein Ausführungsbeispiel einer Gebäudebelüftungsvorrichtung, und
- Fig. 9: eine Explosionsdarstellung des Ausführungsbeispiels der Duftsäule aus Fig. 8.

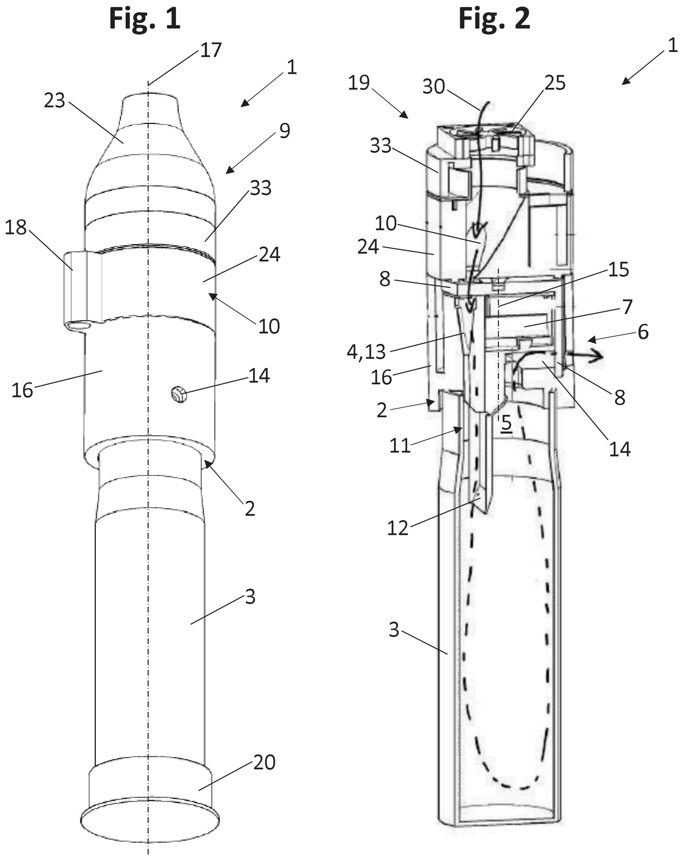

Die Fig. 1 zeigt ein erstes Ausführungsbeispiel einer Vorrichtung 1 zur Abgabe eines Wirkstoffes, insbesondere eines Duftstoffes in einer perspektivischen Darstellung.

Fig. 2 zeigt dasselbe Ausführungsbeispiel an einem Querschnitt.

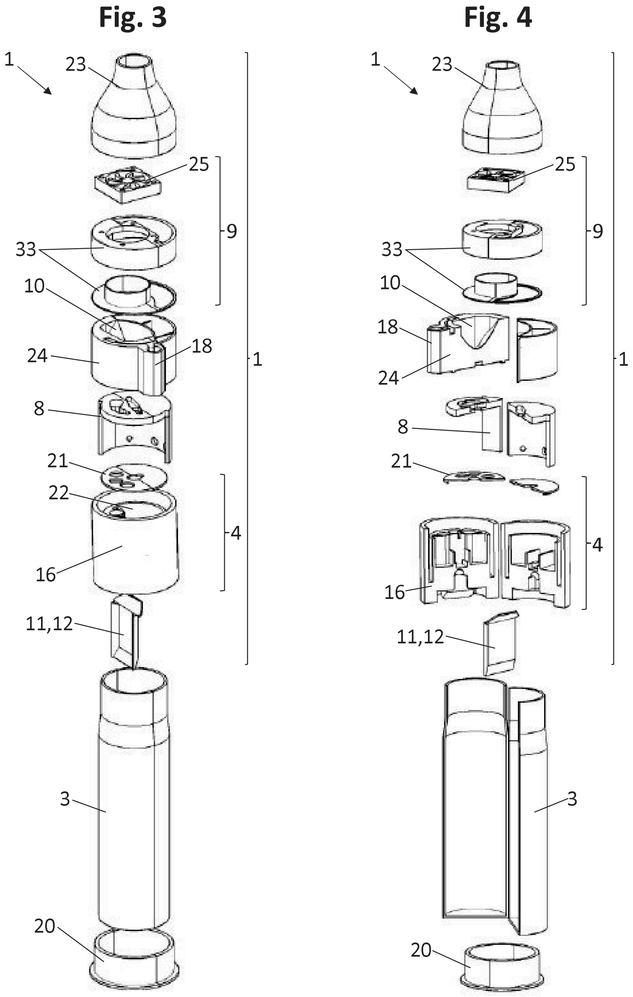

Fig. 3 zeigt das Ausführungsbeispiel der Figuren 1 und 2 in einer Explosionsdarstellung und Fig. 4 zeigt diese Explosionsdarstellung, wobei die einzelnen Komponenten aufgeklappt wurden, sodass das Innere dieser Komponenten ersichtlich ist.

Im Folgenden soll dieses erste Ausführungsbeispiel durch seine Funktion näher erläutert werden, wobei insbesondere Bezug auf die Figuren 1 bis 4 genommen wird.

Dieses Ausführungsbeispiel einer Vorrichtung 1 zur Abgabe eines Wirkstoffes ist dazu ausgebildet, einen Duftstoff an die Umgebungsluft abzugeben.

Dieser Duftstoff wird im Behältnis 3 aufbewahrt.

Das Behältnis 3 kann zusätzlich mit einem Standfuß 20 ausgestattet werden, sodass die Anordnung des Behältnisses 3 mit der Vorrichtung 1 isoliert aufgestellt werden kann.

Die Vorrichtung 1 zur Abgabe des Duftstoffes ist über die Aufnahmevorrichtung 2 mit dem Behältnis 3 verbunden, wobei die Vorrichtung 1 über die Aufnahmevorrichtung 2 an der Öffnung 5 des Behältnisses 3 angebunden wird.

Das Behältnis 3 ist in diesem Ausführungsbeispiel flaschenförmig umgesetzt.

Die Aufnahmevorrichtung 2 ist in diesem Ausführungsbeispiel am Basiskörper 16 der Vorrichtung 1 umgesetzt, wobei durch eine entsprechende Ausnehmung am Basiskörper 16 - genau genommen: welche an einer Stirnseite des zylinderförmigen Basiskörpers positioniert ist 16 -das Behältnis 3 in den Basiskörper 16 eingeschoben werden kann.

Durch eine Presspassung zwischen Aufnahmevorrichtung 2 und Behältnis 3 wird eine formschlüssige Verbindung zwischen Vorrichtung 1 und Behältnis 3 umgesetzt, welche das Innere des Behältnisses 3 über die Vorrichtung 1 gegenüber der Umgebung abschließt.

Die Vorrichtung 1 des Ausführungsbeispiels der Figuren 1 bis 4 umfasst im obersten Abschnitt eine Fördervorrichtung 9.

Diese Fördervorrichtung 9 beinhaltet einen Ventilator 25 sowie die Befestigungskörper 33, welche dazu ausgebildet sind, den Ventilator 25 an der Vorrichtung 1 anzubinden und den durch den Ventilator 25 gebildeten Trägergasstrom 30 der Zuführvorrichtung 4 - genauer gesagt: den ersten Strömungskanälen 13 der Zuführvorrichtung 4 - bereitzustellen.

In diesem Ausführungsbeispiel wird der Trägergasstrom 30 durch den Ventilator 25 der Fördervorrichtung 9 mittels angesaugter Umgebungsluft gebildet.

Diese Umgebungsluft wird von der Fördervorrichtung 9 - genauer gesagt: durch den Ventilator 25 - über die Kappe 23 der Vorrichtung 1 angesaugt.

Diese Kappe 23 ist in der Fig. 2 aus Gründen der Übersichtlichkeit nicht dargestellt. Ebenfalls aus Gründen der Übersichtlichkeit nicht dargestellt in Fig. 2 ist der Standfuß 20.

Nach Bilden dieses Trägergasstroms 30 (in Fig. 2 zur Visualisierung durch die Pfeile dargestellt) wird dieser Trägergasstrom 30 in den Anströmbereich 10 eingeleitet.

Dieser Anströmbereich 10 der Vorrichtung 1 - genau genommen: der Zuführvorrichtung 4 - ist trichterförmig ausgebildet, um den Trägergasstrom 30 zu erfassen und fokussiert als Strahl über die Zuführvorrichtung 4 und die Öffnung 5 des Behältnisses 3 in das Behältnis 3 einzuleiten.

Nach Fokussieren des Trägergasstroms 30 durch den trichterförmigen Anströmbereich wird der Trägergasstrom 30 über die Blende 8 in die ersten Strömungskanäle 13 der Zuführvorrichtung 4 des Basiskörpers 16 geleitet, bevor der Trägergasstrom 30 über die Leiteinrichtung 11 in das Innere des Behältnisses 3 gelangt.

Wie später noch im Detail erläutert wird, kann über die Blende 8 der Vorrichtung 1 der Volumenstrom des Trägergasstroms 30, welcher durch den ersten Strömungskanal 13 strömt, variiert werden.

Nach Durchströmen des Strömungskanals 13 gelangt der Trägergasstrom 30 über die Öffnung 5 des Behältnisses 3 in das Innere des Behältnisses 3, wobei der Trägergasstrom 30 über die Leiteinrichtung 11 - genauer gesagt: das Leitelement 12 - im Inneren des Behältnisses 3 an einen Wandbereich geleitet wird.

Durch dieses Anströmen des Wandbereiches des Behältnisses 3 kann eine vollkommene Durchströmung des Behältnisses 3 erreicht werden, bei welcher (wie durch den strichlierten Pfeil in Fig. 2 dargestellt) sich eine Strömung des Trägergasstroms 30 in einem Wandbereich des Inneren des Behältnisses 3 bis zu einem Flüssigkeitspegel des Wirkstoffes ergibt, an diesem Pegel des Wirkstoffes der Trägergasstrom 30 umgeleitet wird und an einem gegenüberliegenden Wandbereich des Behältnisses 3 retour zur Abführvorrichtung 6 der Vorrichtung 1 geleitet wird, an welcher der mit Wirkstoff beladene Trägergasstrom 30 über die Öffnung 5 des Behältnisses 3 und den zweiten Strömungskanal 14 der Abführvorrichtung 6 an die Umgebung der Vorrichtung 1 freigesetzt wird.

Bei diesem Durchströmen des Inneren des Behältnisses 3 wird der Trägergasstrom 30 durch Verdampfung oder Verdunstung des Wirkstoffes mit dem Wirkstoff beladen, wodurch der Trägergasstrom 30 den Wirkstoff bei Verlassen über die Abführvorrichtung 6 der Vorrichtung 1 den Wirkstoff nach außen trägt.

Der zweite Strömungskanal 14 der Abführvorrichtung 6 der Vorrichtung 1 kann oder ist in seinem Querschnitt durch die Blende 8 veränderbar, wodurch mit Hilfe der Blende 8 ein Volumenstrom des Trägergasstroms 30 durch die Abführvorrichtung 6 veränderbar ist.

Die Blende 8 zur Variation des Volumenstroms des Trägergasstroms 30 ist drehbar um die Rotationsachse 15 im Basiskörper 16 gelagert.

Die Blende 8 ist mit dem Aktuator 7 (in Fig. 2 schematisch dargestellt) verbunden, wobei der Aktuator 7 eine Drehposition der Blende 8 um die Rotationsachse 15 verändern kann.

Somit ist es möglich, durch den Aktuator 7 und die Veränderung der Drehposition der Blende 8 im Basiskörper 16 um die Rotationsachse 15 gleichzeitig sowohl den Volumenstrom des Trägergasstroms 30 durch die Zuführvorrichtung 4 als auch durch die Abführvorrichtung 6 zu verändern.

Der Aktuator 7 ist in diesem Ausführungsbeispiel als elektrischer Rotationsmotor umgesetzt, welcher in der Ausnehmung 22 des Basiskörpers 16 gelagert ist.

Diese Ausnehmung 22 wird zum Schutz des Aktuators 7 vor äußeren Einflüssen durch die Aktuatorabdeckung 21 verschlossen.

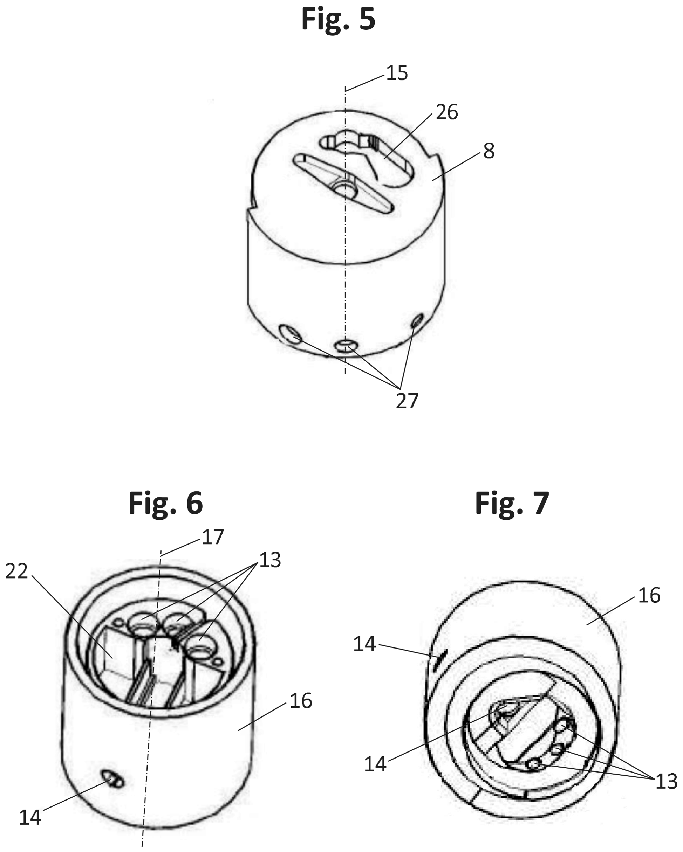

Im Folgenden wird näher auf den Basiskörper 16 und die Blende 8 des Ausführungsbeispiels der Figuren 1 bis 4 unter Bezugnahme auf die Figuren 5 bis 7 eingegangen, wobei Fig. 5 die Blende 8 und die Figuren 6 und 7 den Basiskörper 16 in verschiedenen perspektivischen Darstellungen zeigen.

So ist durch die Fig. 5 ersichtlich, dass die Blende 8 einerseits einen ersten Blendenbereich 26 aufweist und andererseits einen zweiten Blendenbereich 27.

Der erste Blendenbereich 26 ist dazu ausgebildet, je nach Drehstellung der Blende 8 um die Rotationsachse 15 relativ zum Basiskörper 16 einen Volumenstrom des Trägergasstroms 30 durch die Zuführvorrichtung 4 - genauer gesagt: die ersten Strömungskanale 13 der Zuführvorrichtung 4 - zu beeinflussen.

So wird je nach Drehstellung der Blende 8 eine Auswahl oder Teilbereiche der ersten Strömungskanäle 13 verdeckt, wodurch sich der Volumenstrom des Trägergases durch die Zuführvorrichtung 4 verändert.

In gleicher Weise ist die Blende 8 im zweiten Blendenbereich 27 sozusagen als gekrümmte Lochscheibe ausgebildet, welche drei Durchgangsbohrungen mit unterschiedlichen Durchmessern umfasst.

Je nach Drehstellung der Blende 8 gegenüber dem Basiskörper 16 wird somit der zweite Strömungskanal 14 des Basiskörpers 16 durch eine der Bohrungen im zweiten Blendenbereich 27 verringert oder teilweise abgedeckt, womit sich wiederum der Volumenstrom des Trägergasstroms 30 beladen mit Wirkstoff durch den zweiten Strömungskanal 14 beeinflussen lässt.

Es ist zu erkennen, dass durch die um die Rotationsachse 15 drehbar gelagerte Blende 8 ein Volumenstrom durch die Zuführvorrichtung 4 als auch durch die Abführvorrichtung 6 gleichermaßen veränderbar ist.

Auch ein Absperren des ersten Strömungskanals 13 als auch des zweiten Strömungskanals 14 ist durch die Blende 8 möglich, was beispielsweise beim Transport der Vorrichtung 1 wünschenswert sein könnte.

Die Figuren 6 und 7 zeigen unterschiedliche perspektivische Ansichten des Basiskörpers 16.

Es ist zu erkennen, dass die Zuführvorrichtung 4 drei Strömungskanäle 13 aufweist, wobei der mittig angeordnete Strömungskanal 13 den Basiskörper 16 parallel zur Mittelachse 17 durchdringen. Die zwei seitlich angeordneten Strömungskanäle 13 durchsetzen den Basiskörper 16 mit einer leichten Neigung zur Mittelachse 17.

Die 13 Strömungskanäle 13 sind dazu ausgebildet, den Trägergasstrom 30 durch den Basiskörper 16 hindurchzuführen.

Des Weiteren umfasst der Basiskörper 16 den zweiten Strömungskanal 14 der Abführvorrichtung.

Dieser zweite Strömungskanal 14 verbindet eine Öffnung an der Stirnseite mit einer Öffnung an der Zylinderfläche des Basiskörpers 16 und ist dazu ausgebildet, den mit Wirkstoff beladenen Trägergasstrom 30 aus dem Behältnis 3 an die Umgebung der Vorrichtung 1 zu führen.

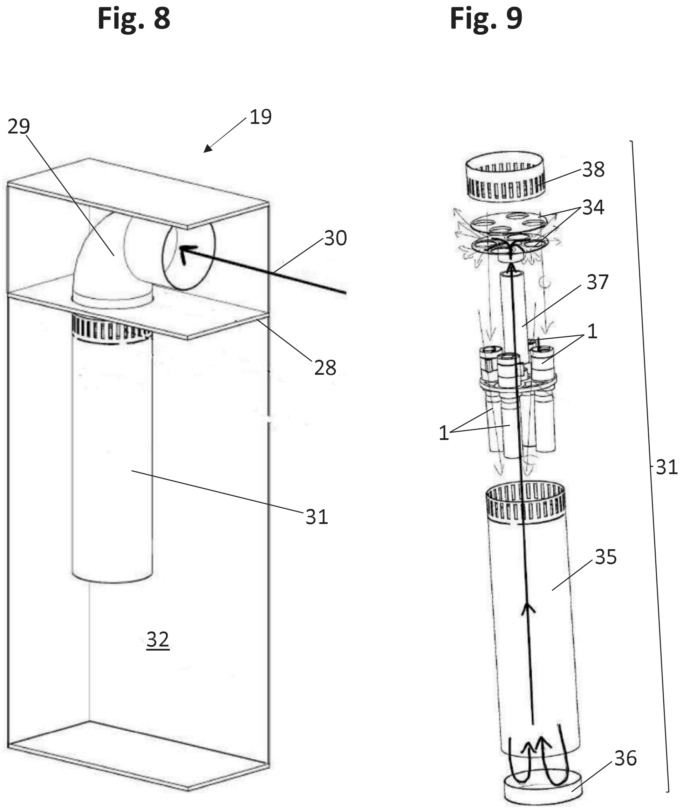

Fig. 8 zeigt ein Ausführungsbeispiel einer Gebäudebelüftungsvorrichtung 19.

Dabei umfasst die Gebäudebelüftungsvorrichtung 19, wie es aus dem Stand der Technik bekannt ist, einen Lüftungsschacht 29, welcher zwischen einer Gebäudedecke und einer Zwischendecke 28 angeordnet ist und dazu ausgebildet ist, zur Belüftung eines Innenraums 32 einen Trägergasstrom 30 zu führen.

Dieser Trägergasstrom 30 ist zumeist eine Frischluft, welche von der Umgebung des Gebäudes entnommen, gefiltert, gegebenenfalls erwärmt oder gekühlt wird und anschließend dem Innenraum 32 über den Lüftungsschacht 29 zugeführt wird.

Dieser Lüftungsschacht 29 ist an der Öffnung zum Innenraum 32 mit einer Duftsäule 31 verbunden, welche Duftsäule 31 zur näheren Erläuterung in Fig. 9 als Explosionsdarstellung dargestellt ist.

So ist ersichtlich, dass der über den Lüftungsschacht 29 zugeführte Trägergasstrom 30 über die Lochscheiben 34 vier Vorrichtungen 1 gemäß dem Ausführungsbeispiel der Figuren 1 bis 4 zuführbar ist, welche Vorrichtungen 1 im Inneren der Duftsäule 31 angeordnet sind. Dieser Trägergasstrom 30 durchläuft diese Vorrichtungen 1 gemäß dem zuvor beschriebenen Ablauf und wird anschließend wieder im Inneren der Aufnahmehülse 35 freigesetzt, in welchem der mit Wirkstoff beladene Trägergasstrom 30 durch den Deckel 36 umgeleitet wird und über das zentrale Rohr 37 zurückgeführt wird, wobei anschließend der Trägergasstrom 30 über das Lochblech 38 in den Innenraum 32 mit Wirkstoff austritt.

### Bezugszeichenliste:

- 1: Vorrichtung
- 2: Aufnahmevorrichtung
- 3: Behältnis
- 4: Zuführvorrichtung
- 5: Öffnung des Behältnisses
- 6: Abführvorrichtung
- 7: Aktuator
- 8: Blende
- 9: Fördervorrichtung
- 10: Anströmbereich
- 11: Leiteinrichtung
- 12: Leitelement
- 13: erster Strömungskanal
- 14: zweiter Strömungskanal
- 15: Rotationsachse
- 16: Basiskörper
- 17: Mittelachse des Basiskörpers
- 18: Befestigungsvorrichtung
- 19: Gebäudebelüftungsvorrichtung
- 20: Standfuß
- 21: Aktuatorabdeckung
- 22: Ausnehmung zur Aufnahme des Aktuators
- 23: Kappe
- 24: Anströmkörper
- 25: Ventilator
- 26: erster Blendenbereich
- 27: zweiter Blendenbereich
- 28: Zwischendecke
- 29: Lüftungsschacht
- 30: Trägergasstrom
- 31: Duftsäule
- 32: Innenraum
- 33: Befestigungskörper
- 34: Lochscheibe
- 35: Hülse
- 36: Deckel
- 37: Rohr
- 38: Lochblech

## Patentansprüche

1. Vorrichtung zur Abgabe eines Wirkstoffes, insbesondere eines Duftstoffes, umfassend
- eine Aufnahmevorrichtung (2) zur Befestigung der Vorrichtung (1) an einer Öffnung (5) eines Behältnisses (3), vorzugsweise einer Flasche, zur Aufnahme des Wirkstoffes,
- eine Zuführvorrichtung (4), welche Zuführvorrichtung (4) dazu ausgebildet ist, einen Trägergasstrom (30), vorzugsweise einen Luftstrom, über die Öffnung (5) des Behältnisses (3) in das Behältnis (3) zu leiten, und
- eine Abführvorrichtung (6), welche Abführvorrichtung (6) dazu ausgebildet ist, ein mit Wirkstoff beladenen Trägergasstrom (30) über die Öffnung (5) des Behältnisses (3) aus dem Behältnis (3) abzuführen,
wobei wenigstens eine mit einem Aktuator (7) verbundene Blende (8) vorgesehen ist, wobei durch den Aktuator (7) und die damit verbundene wenigstens eine Blende (8) ein Volumenstrom des Trägergasstroms (30) durch die Zuführvorrichtung (4) und/oder die Abführvorrichtung (6) veränderbar ist.

2. Vorrichtung nach Anspruch 1, wobei wenigstens eine Fördervorrichtung (9) zum Erzeugen des Trägergasstroms (30) über die Zuführvorrichtung (4) in das Behältnis (3) vorgesehen ist, vorzugsweise wobei die wenigstens eine Fördervorrichtung (9) zumindest einen Ventilator (25) umfasst, besonders bevorzugt welche in Strömungsrichtung stromaufwärts der Zuführvorrichtung (4) angeordnet ist.

3. Vorrichtung nach einem der vorhergehenden Ansprüche, wobei die Zuführvorrichtung (4) einen Anströmbereich (10) aufweist, welcher - vorzugsweise trichterförmig ausgebildete - Anströmbereich (10) dazu ausgebildet ist, den Trägergasstrom (30) zu erfassen und fokussiert als Strahl über die die Öffnung (5) des Behältnisses (3) in das Behältnis (3) einzuleiten.

4. Vorrichtung nach wenigstens einem der vorhergehenden Ansprüche, wobei die Zuführvorrichtung (4) zumindest eine Leiteinrichtung (11) aufweist, welche zumindest eine Leiteinrichtung (11) dazu ausgebildet ist, den in das Behältnis (3) eingeleiteten Trägergasstrom (30) auf einen Wirkstoffpegel im Behältnis (3) zu leiten.

5. Vorrichtung nach dem vorhergehenden Anspruch, wobei die zumindest eine Leitvorrichtung (11) ein Leitelement (12), vorzugsweise ein Leitblech, aufweist, welches Leitelement (12) an der Aufnahmevorrichtung (2) hervorsteht, sodass das Leitelement (12) in einem Befestigungszustand der Vorrichtung (1) an dem den Wirkstoff tragenden Behältnis (3) über die Öffnung (5) des Behältnisses (3) in das Innere des Behältnisses (3) ragt.

6. Vorrichtung nach wenigstens einem der vorhergehenden Ansprüche, wobei die wenigstens eine Blende (8) als Lochscheibe ausgebildet ist, welche Lochscheibe durch eine oder eine Vielzahl an Öffnungen - vorzugsweise Bohrungen mit unterschiedlichen Durchmessern - dazu ausgebildet ist, einen Strömungsquerschnitt eines den Trägergasstrom (30) leitenden Strömungskanals (13, 14) der Zuführvorrichtung (4) und/oder der Abführvorrichtung (6), je nach Relativstellung der Lochscheibe zum Strömungskanal (13, 14), zu variieren.

7. Vorrichtung nach dem vorhergehenden Anspruch, wobei die Lochscheibe drehbar um eine Rotationsachse (15) gelagert ist und der, vorzugsweise als rotatorischer Motor, besonders bevorzugt rotatorischer Elektromotor, ausgebildete, Aktuator (7) dazu ausgebildet ist, die Lochscheibe rotatorisch um die Rotationsachse (15) anzutreiben.

8. Vorrichtung nach wenigstens einem der vorhergehenden Ansprüche, wobei die Vorrichtung (1) einen, vorzugsweise eine Mittelachse (17) aufweisenden zylinderförmigen, Basiskörper (16) aufweist, welcher Basiskörper (16):
- einen ersten Strömungskanal (13) der Zuführvorrichtung (4) aufweist, vorzugsweise welcher erste Strömungskanal (13) der Zuführvorrichtung (4) den Basiskörper (16) parallel zur Mittelachse (17) durchdringt, und/oder
- einen zweiten Strömungskanal (14) der Abführvorrichtung (6) aufweist, vorzugweise welcher zweite Strömungskanal (14) der Abführvorrichtung (6) eine Öffnung an einer Stirnseite des Basiskörpers (16) mit einer Öffnung an einer Zylinderfläche des Basiskörpers (16) verbindet, und/oder
- einstückig mit der Aufnahmevorrichtung (2) zur Befestigung der Vorrichtung (1) an einer Öffnung (5) eines Behältnisses (6), vorzugsweise an einer Stirnseite des Basiskörpers (16), ausgebildet ist, und/oder
- dazu ausgebildet ist, die wenigstens eine Blende (8) aufzunehmen, und vorzugsweise die wenigstens eine Blende (8) rotatorisch zu lagern, und/oder
- dazu ausgebildet ist, den Aktuator (7) aufzunehmen und/oder zu lagern, und/oder
- wenigstens eine Befestigungsvorrichtung (18) zur Befestigung der Vorrichtung (1) an einem externen Objekt aufweist.

9. Vorrichtung nach wenigstens einem der vorhergehenden Ansprüche, wobei eine mit dem Aktuator (7) signalleitend verbundene Steuer- oder Regelvorrichtung vorgesehen ist, welche dazu ausgebildet ist, durch Steuern oder Regeln des Aktuators (7) den Volumenstrom des Trägergasstroms (30) durch die Zuführvorrichtung (4) und/oder die Abführvorrichtung (6) zu variieren.

10. Vorrichtung nach dem vorhergehenden Anspruch, wobei die Steuer- oder Regelvorrichtung eine Eingabeschnittstelle aufweist oder mit einer solchen signalleitend verbunden ist, wobei die Steuer- oder Regelvorrichtung dazu ausgebildet ist, eine Anpassung des Volumenstroms unter Berücksichtigung eines über die Eingabeschnittstelle bereitgestellten Signals vorzunehmen, welches Signal mittels der Eingabeschnittstelle über eine Datenübertragungsverbindung entgegengenommen werden kann.

11. Anordnung einer Vorrichtung (1) nach wenigstens einem der vorhergehenden Ansprüche und einem Behältnis (3) zur Aufnahme des Wirkstoffes, wobei die Vorrichtung über die Aufnahmevorrichtung (2) an einer Öffnung (5) des Behältnisses (3) befestigt ist.

12. Gebäudebelüftungsvorrichtung (19) umfassend eine Vorrichtung (1) nach wenigstens einem der Ansprüche 1 bis 10 oder eine Anordnung nach Anspruch 11.
